# EUROPÄISCHE PATENTSCHRIFT

(11) **EP 1 854 871 B1**
(45) Veröffentlichungstag und Bekanntmachung des Hinweises auf die Patenterteilung: **22.10.2008**
(21) Anmeldenummer: 07004619.8
(22) Anmeldetag: 06.03.2007
(51) Int. Cl.: C12M 1/00, C12M 1/02

(54) **Schrank sowie Bearbeitungsverfahren**
Cabinet and processing method
Armoire tout comme procédé de traitement

(30) Priorität: 09.05.2006 DE 102006021852
(43) Veröffentlichungstag der Anmeldung: 14.11.2007
(73) Patentinhaber: Infors AG, 4103 Bottmingen (CH)
(72) Erfinder: Hawrylenko, Alexander, 4103 Bottmingen (CH)
(74) Vertreter: Säger, Manfred

(56) Entgegenhaltungen:
- EP-A- 0 339 824
- EP-A- 0 569 214
- EP-A- 1 626 082
- EP-A1- 1 445 307
- WO-A-20/06074569
- GB-A- 1 327 632
- US-A- 3 002 895
- US-A- 5 735 587

## Beschreibung

Die Erfindung betrifft einen gattungsgemässen, mittels einer Tür geschlossene Schrank nach dem Oberbegriff des Hauptanspruchs.

Solche Schränke sind als temperier- sowie begasbare Inkubatoren oder Reaktoren in einer Vielzahl von Ausführungsformen bekannt. Die auf deren Tablaren angeordneten Behältnisse werden mit ihrem Inhalt im Inneren des Schranks zumeist bei bestimmten Umgebungsbedingungen wie Luftdruck, Feuchtigkeit, Temperatur oder beim Vorhandensein bestimmter Gase geschüttelt. Von Zeit zu Zeit müssen die Flüssigkeiten und/oder Kulturen in den Behältnissen verfahrenstechnisch bearbeitet werden. Dazu muss der Schüttelantrieb angehalten, die Tür des Schranks geöffnet und in den meisten Fällen die Behältnisse von Hand herausgeholt oder in neueren bekannten Ausführungsformen das Tablar mit den Behältnissen manuell entkoppelt und anschliessend herausgezogen werden. Erst dann können die einzelnen Behältnisse geöffnet und, je nach Inhalt und/oder Zweck, verschieden verfahrenstechnisch bearbeitet, z.B. pipettiert werden. Danach müssen die einzelnen Behältnisse wieder von Hand geschlossen, in den Schrank eingesetzt oder mit dem Tablar eingeschoben, dieses an den Schüttelantrieb angekoppelt und die Tür geschlossen werden; erst dann kann der Schüttelantrieb wieder eingeschaltet werden, was sich insgesamt als aufwändig darstellt.

Der Erfindung liegt die Aufgabe zugrunde, einen gattungsgemässen Schrank zum automatischen verfahrenstechnischen Bearbeiten auszugestalten.

Diese Aufgabe wird erfindungsgemäss bei einem gattungsgemässen Schrank sowie Bearbeitungsverfahren nach dem Oberbegriff des Sach- sowie Verfahrenshauptanspruchs erfindungsgemäss durch deren kennzeichnende Merkmale gelöst.

Mit der Erfindung ist es also erstmalig möglich, die in dem geschlossenen temperierten Schrank in Form eines Inkubators oder Reaktors enthaltenen Proben in den Behältnissen nicht nur auf dem Tablar automatisch zu schütteln, sondern es ist auch möglich, den erfindungsgemäss ausgestalteten Schrank in die erfindungsgemässe automatische verfahrenstechnische Bearbeitung mit einzubeziehen, und das Tablar selbsttätig von dem Innern des Schrankes in eine solche Position ausserhalb desselben automatisch zu verbringen, dass sich die darauf befindlichen Behältnisse in einer wohldefinierten Lage befinden, wodurch es z.B. einem Roboter ermöglicht wird, die verschiedenen einzelnen Behältnisse, je nach Bedarf und/oder Programm erforderlichenfalls zu öffnen und z.B. mit einer vorherbestimmten Menge einer bestimmten Menge einer ersten Flüssigkeit, z.B. Nährmedium und andere Behältnisse mit einer anderen Menge einer zweiten Flüssigkeit zu pipettieren. Nachdem dieses Pipettieren abgeschlossen ist, kann das Tablar mit den Behältnissen wieder automatisch in das Innere des Schranks gebracht, wieder an den Schüttelantrieb angekuppelt und die Tür verschlossen werden, wonach auch der Schüttelantrieb automatisch wieder eingeschaltet werden kann.

Zweckmässige Ausgestaltungen und Weiterbildungen der Erfindung sind in den Unteransprüchen gekennzeichnet.

Ein Ausführungsbeispiel der Erfindung wird nachfolgend unter Bezugnahme auf die Zeichnung näher erläutert. In dieser zeigt:
- Figur 1: einen erfindungsgemässsen Schrank, in schematischer, perspektivischer Darstellung
- Figur 2: den Schrank gemäss Figur 1, im schematischen Querschnitt und
- Figur 3: ein Schaltbild der erfindungsgemässen Ablaufsteuerung.

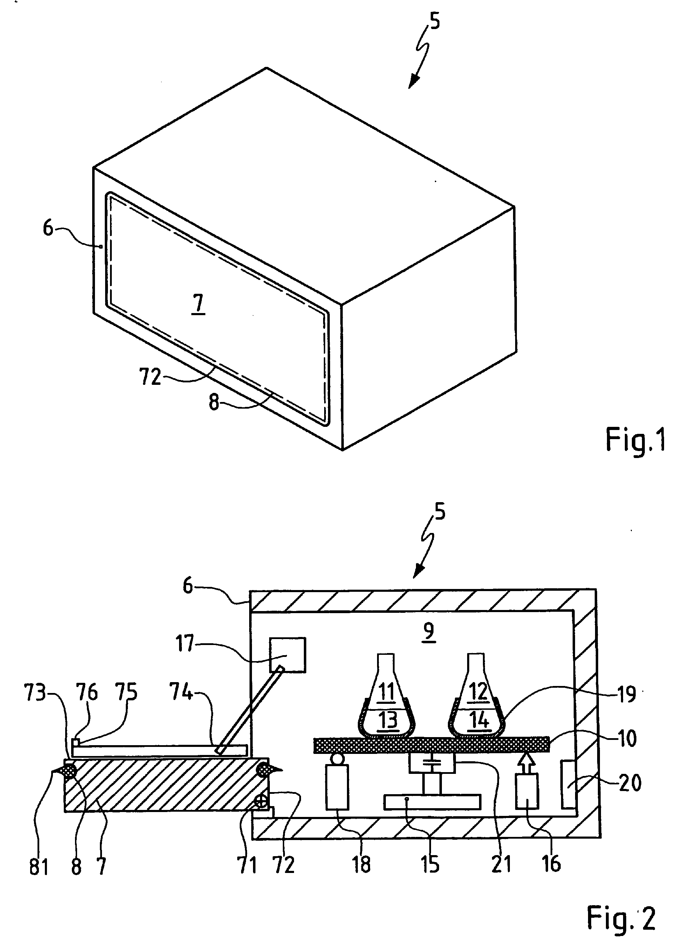

Der erfindungsgemässe als geschlossener Schrank 5 ausgebildete Inkubator, Reaktor od. dgl. ist im wesentlichen quaderförmig ausgebildet und weist beim wiedergegbenen Ausführungsbeispiel an seiner Vorderseite 6 eine als um eine horizontale Achse 71 im Bereich ihrer unteren Kante 72 schwenkbare Klappe 7 ausgebildete Tür auf, längs deren Rand eine umlaufende Dichtung 8, wie gestrichelt in Figur 1 angedeutet, mit einer vorspringenden Dichtkante 81 in einer umlaufenden Nut 76 der Klappe 7 angeordnet ist.

Im Inneren 9 des Schranks 5, das in Figur 2 gezeigt ist, ist ein Tablar 10 mit zwei schematisch angedeuteten, darauf befindlichen Behältnissen 11,12 angeordnet, die jeweils eine zu schüttelnde Flüssigkeit 13 bzw. 14 mit oder ohne Kultur aufweisen und mittels einer elastisch nachgiebigen Klemmeinrichtung 19 an dem Tablar gehalten sind.

Das Tablar 10 ist über eine Kupplungseinrichtung 21 an den darunter angeordneten Schüttelantrieb 15 angetrieben und kann davon abgekuppelt oder daran angekuppelt, d.h. gelöst werden, so dass das Tablar andere Bewegungsabläufe als den Schüttelbetrieb ausführen kann. Das Tablar 10 kann von einer insgesamt mit 16 bezeichneten Anhaltepositioniereinrichtung zum Beenden des Schüttelbetriebs in einer vorherbestimmbaren definierten Position angehalten werden. Zum Öffnen nach dem Anhalten des Tablars 10 in der definierten Position weist die Klappe 7 einen Bewegungsantrieb 17 auf, der auch zum Schliessen der Tür nach dem Hineinziehen des Tablars dient.

Ferner ist die Klappe 7 mit einer ebenen Innenseite 73 und auf dieser mit einer Führung 74 für das Tablar 10 versehen. Die ebene Innenseite 73 bildet im geöffnetem Zustand der Klappe 7 eine horizontale Fläche, wobei die horizontale Achse 71 der Klappe 7 so angeordnet ist, dass sich die Innenseite 73 der geöffneten Klappe 7 zu der Unterseite des Tablars 10 so fluchtet, dass dieses längs der Führung 74 an der Innenseite 73 der Klappe 7 bis in eine definierte Endstellung mit einer Arretierung 75 herausschiebbar ist, die auch einen ein elektrisches Signal abgebenden Endschalter 76 aufweist.

Das Tablar 10 ist zu dessen Herausschieben aus dem Inneren 9 des Schranks 5 sowie zum wieder Hineinziehen dahin längs der Führung 74 ferner mit einem Verschiebeantrieb 18 aus dem Schrank 5 bei geöffneter Klappe 7 versehen. Es versteht sich von selbst, dass das Tablar 10, der Schüttelantrieb 15 sowie die Kupplungseinrichtung 21 und die Anhaltepositioniereinrichtung 16 voneinander entkuppelbar sind, so dass es mittels des Verschiebeantriebes 18 längs der Führung 74 aus dem Inneren 9 des Schranks 5 herausfahrbar ist.

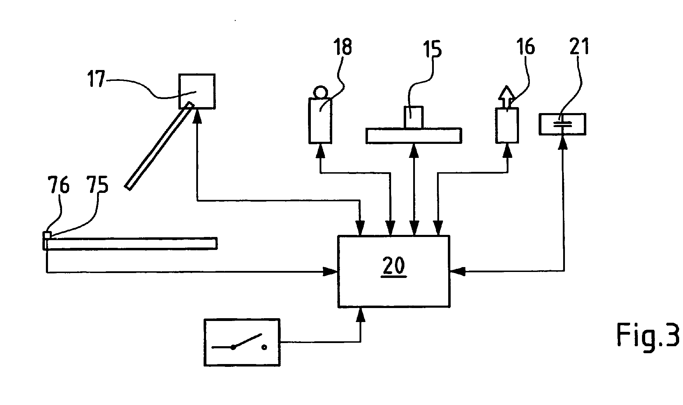

In Figur 3 ist eine erfindungsgemässe Ablaufsteuerung 20 gezeigt, die mit dem Schüttelantrieb 15, mit der Kupplungseinrichtung 21, mit der Anhaltepositioniereinrichtung 16, mit dem Bewegungsantrieb 17 und mit dem Verschiebeantrieb 18 jeweils verbunden ist und zum Öffnen der Klappe 7 nach dem Anhalten des Tablars 10 in der definierten Position, zum Herausschieben des Tablars 10 aus dem Schrank 5 bei geöffneter Klappe 7 bis zur Betätigung des Endschalters 76 sowie zum Schliessen der Klappe 7 nach dem Hineinziehen des Tablars 10 durch den Verschiebeantrieb 18, zum Herausschieben sowie Hineinziehen des Tablars aus dem Schrank bei geöffneter Tür vorgesehen ist.

Der erfindungsgemässe Schrank 5 sowie die Ablaufsteuerung 20 ermöglichen dabei ein automatisches Verfahren zum Bearbeiten der zu schüttelnden Flüssigkeiten 13,14 (mit oder ohne Kultur), wobei folgende Verfahrensschritte selbsttätig ablaufen, nämlich
- dass das Tablar 10 nach dem Beenden des Schüttelbetriebs mittels der Anhaltepositioniereinrichtung 16 in einer vorherbestimmbaren definierten Position angehalten wird;
- dass das Tablar 10 dann mittels der Kupplungseinrichtung 21 von dem Schüttelantrieb 15 abgekuppelt wird;
- dass danach die Klappe 7 geöffnet und das Tablar 10 mit dem Verschiebeantrieb 18 längs der Führung 74 aus dem Schrank bis in eine definierte Endstellung herausgeschoben und dort arretiert;
- dass danach die Behältnisse 11,12 automatisch verfahrenstechnisch, vorzugsweise von einem Roboter bearbeitet, z.B. pipettiert werden;
- dass das Tablar 10 nach der verfahrenstechnischen Bearbeitung von dem Verschiebeantrieb 18 wieder in den Schrank 5 hineingezogen wird;
- dass das Tablar 10 anschliessend mittels der Kupplungseinrichtung 21 an den Schüttelantrieb 15 angekuppelt wird,
- wonach die Klappe 7 geschlossen und das Tablar 10 wieder von dem Schüttelantrieb 15 angetrieben und damit die Behältnisse 11,12 geschüttelt werden.

## Patentansprüche

1. Mittels einer Tür (7) geschlossener Schrank (5) in Form eines Inkubators oder Reaktors, mit einem darin angeordneten, mittels eines Schüttelantriebes (15) angetriebenes, längs einer Führung (74) aus dem Schrank (5) herausfahrbares Tablar (10) und mit zumindest einem darauf in einer Klemmeinrichtung (19) gehaltenen Behältnis (11,12), das eine zu schüttelnde Flüssigkeit (13,14) mit oder ohne Kultur aufweist, **dadurch gekennzeichnet, dass** die Tür das Innere (9) des Schranks dicht abschliessend ausgebildet ist, dass der Schüttelantrieb (15) mit einer Anhaltepositioniereinrichtung (16) zum Beenden des Schüttelbetriebs in einer vorherbestimmbaren definierten Position des Tablars (10) ausgebildet ist, dass die TÜr (7) einen Bewegungsantrieb (17) zu ihrem Öffnen nach dem Anhalten des Tablars (10) in der definierten Position sowie Schliessen der Tür (7) nach dem Hineinziehen des Tablars (10) aufweist, dass das Tablar (10) mit einem Verschiebeantrieb (18) zu dessen Herausschieben sowie Hineinziehen längs der Führung (74) aus dem Schrank (5) bei geöffneter Tür (7) versehen ist, dass eine Kupplungseinrichtung (21) zum Abkuppeln des Schüttelantriebes (15) von dem Tablar (10) vor dessen Herausschieben und zu dessen Ankuppeln an den Schüttelantrieb (15) nach dem Hineinziehen des Tablars in das Innere (9) des Schrankes vor Beginn des Schüttelbetriebs vorgesehen ist und dass in dem Schrank (5) eine mit der Anhaltepositioniereinrichtung (16), mit dem Schüttelantrieb (15), mit dem Bewegungsantrieb (17), mit der Kupplungseinrichtung (21) und mit dem Verschiebeantrieb (18) jeweils verbundene Ablaufsteuerung (20) zum Öffnen der Tür (7) mittels des Bewegungsantriebs (17) nach dem Anhalten und dem Entkuppeln des Tablars (10) von dem Schüttelantrieb (15) in der definierten Position, zum Schliessen der Tür (7) nach dem Hineinziehen des Tablars (10) und zum Ankuppeln des Tablars (10) an den Schüttelantrieb (15) mittels der Kupplungseinrichtung (21) vor Beginn des Schüttelbetriebs und zum Herausschieben sowie Hineinziehen des Tablars (10) aus dem Schrank (5) bei geöffneter Tür (7) mittels des Verschiebeantriebes (18) vorgesehen ist.

2. Schrank nach Anspruch 1, **dadurch gekennzeichnet, dass** die Tür als um eine horizontale Achse (71) im Bereich ihrer unteren Kante (72) schwenkbare Klappe (7) ausgebildet ist und dass diese eine das Innere (9) des Schranks dicht abschliessende, an deren Rand umlaufende Dichtung (8) aufweist.

3. Schrank nach Anspruch 2, **dadurch gekennzeichnet, dass** der Schrank (5) im wesentlichen quaderförmig ausgebildet ist und sich die Klappe (7) über dessen Vorderseite (6) erstreckt.

4. Schrank nach Anspruch 3, **dadurch gekennzeichnet, dass** die Klappe (7) eine ebene Innenseite (73) und diese die Führung (74) für das aus dem Schrank (5) herausschiebbare Tablar (10) aufweist.

5. Schrank nach Anspruch 3 oder 4, **dadurch gekennzeichnet, dass** die Innenseite (73) der Klappe (7) in deren geöffnetem Zustand eine horizontale Fläche bildet und dass die horizontale Achse (71) der Klappe (7) so angeordnet ist, dass die Innenseite (73) der geöffneten Klappe (7) zur Unterseite des Tablars (10) so fluchtet, dass dieses längs der Führung (74) an der Innenseite der Klappe bis in eine definierte Endstellung herausschiebbar ist.

6. Schrank nach Anspruch 3, **dadurch gekennzeichnet, dass** die Führung (74) an der Innenseite (73) der Klappe (7) in der definierten Endstellung eine Arretierung und/oder einen elektrischen Endschalter (76) für das Tablar (10) aufweist.

7. Schrank nach einem der Ansprüche 2 bis 5, **dadurch gekennzeichnet, dass** die an dem Rand der Klappe (7) umlaufende Dichtung (8) eine geschlossene Dichtkante (81) aufweist.

8. Verfahren zum Bearbeiten der in einem mittels einer Tür (7) geschlossener Schrank (5) in Form eines Inkubators oder Reaktors befindlichen zumindest einem darauf in einer Klemmeinrichtung (19) gehaltenen Behältnis (11,12), das eine zu schüttelnde Flüssigkeit (12,13) mit oder ohne Kultur aufweist, wobei der Schrank (5) ein darin angeordnetes, mittels eines Schüttelantriebes (15) angetriebenes, längs einer Führung (74) aus dem Schrank (5) herausziehbares Tablar (10) mit dem zumindest darauf befindlichen einen Behältnis (11,12), **dadurch gekennzeichnet, dass** eine folgende Verfahrensschritte bewirkende Ablaufsteuerung (20) vorgesehen ist, dass das Tablar (10) nach dem Beenden des Schüttelbetriebs mittels einer Anhaltepositioniereinrichtung (16) in einer vorherbestimmbaren definierten Position angehalten wird, dass der Schüttelantrieb (15) vor dem Herausschieben des Tablars (10) von diesem mittels einer Kupplungseinrichtung (21) abgekuppelt wird, dass danach und nach Öffnen der Tür (7) das Tablar (10) mit einem Verschiebeantrieb (18) längs der Führung (74) aus dem Schrank (5) bei geöffneter Tür (7) bis in eine definierte Endstellung herausgeschoben sowie dort arretiert und danach das zumindest eine Behältnis (11,12) automatisch verfahrenstechnisch, vorzugsweise von einem Roboter bearbeitet wird.

9. Verfahren nach Anspruch 8, **dadurch gekennzeichnet, dass** das Tablar (10) nach der verfahrenstechnischen Bearbeitung von dem Verschiebeantrieb (18) wieder in das Innere (9) des Schranks (5) hineingezogen wird.

10. Verfahren nach Anspruch 8 oder 9, **dadurch gekennzeichnet, dass** das Tablars (10) nach dem Hineinziehen in das Innere (9) des Schrankes (5), dem Schliessen der Tür (7) und vor Beginn des Schüttelbetriebs mittels der Kupplungseinrichtung (21) an den Schüttelantrieb (15) angekuppelt wird.

11. Verfahren nach einem der Ansprüche 8 bis 10, **dadurch gekennzeichnet, dass** nach dem Ankuppeln des Tablars (10) an den Schüttelantrieb (15) dieser von der Ablaufsteuerung (20) wieder in den Schüttelbetrieb versetzt und das zumindest eine Behältnis (11,12) wieder geschüttelt wird.

## Claims

1. Cabinet (5) in the form of an incubator or reactor, which is closed by means of a door (7) and contains a pull-out shelf (10),
which is driven by means of a shaker drive (15) and capable of being extended out of the cabinet (5) along a guide (74),
with at least one container (11, 12), which is held on the latter in a clamping device (19) and contains a liquid (13, 14) with or without culture to be shaken,
**characterised in that**
the door is designed to close the interior (9) of the cabinet in a tightly sealed manner,
that the shaker drive (15) is designed with a stopping-and-positioning device (16) for the termination of the shaking operation at a predeterminable, defined position of the pull-out shelf (10),
that the door (7) provides a movement drive (17) in order to open it after the stopping of the pull-out shelf (10) in the defined position and in order to close the door (7) after the retraction of the pull-out shelf (10),
that the pull-out shelf (10) is provided with a displacement drive (18) in order to extend it and retract it along the guide (74) out of/into the cabinet (5) when the door (7) is opened,
that a coupling device (21) is provided for uncoupling the shaker drive (15) from the pull-out shelf (10) before it is extended and for coupling it to the shaker drive (15) after the retraction of the pull-out shelf into the interior (9) of the cabinet before the beginning of the shaking operation,
and that a procedural control unit (20) connected respectively to the stopping-and-positioning device (16), the shaker drive (15), the movement drive (17), the coupling device (21) and the displacement drive (18) is provided in the cabinet (5)
in order to open the door (7) by means of the movement drive (17) after the stopping and uncoupling of the pull-out shelf (10) from the shaker drive (15) in the defined position;
in order to close the door (7) after the retraction of the pull-out shelf (10);
in order to couple the pull-out shelf (10) to the shaker drive (15) by means of the coupling device (21) before the beginning of the shaking operation; and in order to extend and retract the pull-out shelf (10) out of/into the cabinet (5) by means of the displacement drive (18), when the door (7) is opened.

2. Cabinet according to claim 1
**characterised in that**
the door is designed as a flap (7) rotatable about a horizontal axis (71) in the region of its lower edge (72), and that this flap provides a seal (8) extending around its edge and closing the interior (9) of the cabinet in a tightly-sealed manner.

3. Cabinet according to claim 2,
**characterised in that**
the cabinet (5) is designed substantially as a cuboid, and the flap (7) extends over its front side (6).

4. Cabinet according to claim 3,
**characterised in that**
the flap (7) comprises a flat internal side (73), which provides the guide (74) for the pull-out shelf (10), which is capable of being extended from the cabinet (5).

5. Cabinet according to claim 3 or 4,
**characterised in that**
the internal side (73) of the flap (7) forms a horizontal surface in its open condition and that the horizontal axis (71) of the flap (7) is disposed such that the internal side (73) of the open flap (7) is aligned with the underside of the pull-out shelf (10) in such a manner that the latter can be displaced up to a defined end position along the guide (74) on the internal side of the flap.

6. Cabinet according to claim 3,
**characterised in that,**
at the defined end position, the guide (74) on the internal side (73) of the flap (7) provides a stop and/or an electrical end switch (76) for the pull-out shelf (10).

7. Cabinet according to any one of claims 2 to 5,
**characterised in that**
the seal (8) extending around the edge of the flap (7) provides a closed sealing edge (81).

8. Method for processing the at least one container (11, 12),
which is disposed in a cabinet (5) designed in the form of an incubator or reactor and closed by means of a door (7),
which is held in a clamping device (19) and
which contains a liquid (12, 13) to be shaken with or without culture,
wherein the cabinet (5) contains a pull-out shelf (10), which is driven by means of a shaker drive (15) and which is capable of being extended from the cabinet (5) along a guide (74) with the at least one container (11, 12) disposed upon it,
**characterised in that**
a procedural control unit (20), which brings about the following procedural stages, is provided:
the pull-out shelf (10) is stopped in a predeterminable, defined position by means of a stopping-and-positioning device (16) after the termination of the shaking operation;
before the extension of the pull-out shelf (10), the shaker drive (15) is uncoupled from the latter by means of a coupling device (21);
after this and after the opening of the door (7), the pull-out shelf (10) is extended, with the door (7) open, by a displacement drive (18) along the guide (74) out of the cabinet (5) into a defined end position, where it is locked, and following this, the at least one container (11, 12) is automatically subjected to a technical process, preferably by a robot.

9. Method according to claim 8,
**characterised in that,**
after the technical process, the pull-out shelf (10) is retracted into the interior (9) of the cabinet (5) by the displacement drive (18).

10. Method according to claim 8 or 9,
**characterised in that,**
after retraction into the interior (9) of the cabinet (5), after the closing of the door (7) and before the beginning of the shaking operation, the pull-out shelf (10) is coupled to the shaker drive (15) by means of the coupling device (21).

11. Method according to any one of claims 8 to 10,
**characterised in that,**
after the pull-out shelf (10) has been coupled to the shaker drive (15), the shaker drive is again placed into the shaker mode by the procedural control unit (20), and the at least one container (11, 12) is again shaken.

## Revendications

1. Armoire (5) sous forme d'un incubateur ou d'un réacteur, fermée au moyen d'une porte (7) et comprenant une tablette (10) disposée à l'intérieur, entraînée au moyen d'un entraînement agitateur-secoueur (15) et pouvant être sortie de l'armoire (5) le long d'un guide (74), et au moins un récipient (11, 12) maintenu dessus dans un dispositif de serrage (19), qui présente un liquide (13, 14) à agiter, avec ou sans culture, **caractérisée en ce que** la porte est réalisée de façon à fermer l'intérieur (9) de l'armoire de façon étanche, **en ce que** l'entraînement agitateur-secoueur (15) est conçu avec un dispositif de positionnement d'arrêt (16) pour terminer l'opération d'agitation dans une position définie prédéterminable de la tablette (10), **en ce que** la porte (7) présente un entraînement de déplacement (17) pour son ouverture après l'arrêt de la tablette (10) dans la position définie et pour la fermeture de la porte (7) après la rentrée de la tablette (10) à l'intérieur, **en ce que** la tablette (10) est dotée d'un entraînement de coulissement (18) pour sa sortie vers l'extérieur ou sa rentrée à l'intérieur le long du guide (74) hors ou au sein de l'armoire (5) avec la porte (7) ouverte, **en ce qu'**un dispositif d'accouplement (21) est prévu pour le désaccouplement de l'entraînement agitateur-secoueur (15) de la tablette (10) avant sa sortie vers l'extérieur et pour son accouplement à l'entraînement agitateur-secoueur (15) après la rentrée de la tablette à l'intérieur (9) de l'armoire avant le début de l'opération d'agitation, et **en ce que** dans l'armoire (5) est prévue une commande séquentielle (20) reliée respectivement au dispositif de positionnement d'arrêt (16), à l'entraînement agitateur-secoueur (15), à l'entraînement de déplacement (17), au dispositif d'accouplement (21) et à l'entraînement de coulissement (18) pour l'ouverture de la porte (7) au moyen de l'entraînement de déplacement (17) après l'arrêt et le désaccouplement de la tablette (10) de l'entraînement agitateur-secoueur (15) dans la position définie, pour la fermeture de la porte (7) après la rentrée à l'intérieur de la tablette (10) et pour l'accouplement de la tablette (10) à l'entraînement agitateur-secoueur (15) au moyen du dispositif d'accouplement (21) avant le début de l'opération d'agitation et pour la sortie vers l'extérieur et la rentrée à l'intérieur de la tablette (10) hors et au sein de l'armoire (5) avec la porte (7) ouverte au moyen de l'entraînement de coulissement (18).

2. Armoire selon la revendication 1, **caractérisée en ce que** la porte est conçue sous forme de volet (7) pouvant pivoter autour d'un axe (71) horizontal dans la zone de son arête (72) inférieure, et **en ce que** ledit volet présente un joint (8) s'étendant le long de son bord et fermant l'intérieur (9) de l'armoire de façon étanche.

3. Armoire selon la revendication 2, **caractérisée en ce que** l'armoire (5) est conçue essentiellement avec une forme de parallélépipède et le volet (7) s'étend sur sa face avant (6).

4. Armoire selon la revendication 3, **caractérisée en ce que** le volet (7) présente une face intérieure (73) plane et celle-ci présente le guide (74) pour la tablette (10) pouvant être sortie de l'armoire (5).

5. Armoire selon la revendication 3 ou 4, **caractérisée en ce que** la face intérieure (73) du volet (7) forme une surface horizontale dans son état ouvert et **en ce que** l'axe (71) horizontal du volet (7) est disposé de telle sorte que la face intérieure (73) du volet (7) ouvert est aligné avec la face inférieure de la tablette (10) de telle sorte que ladite tablette peut être sortie le long du guide (74) sur la face intérieure du volet jusqu'à atteindre une position finale définie.

6. Armoire selon la revendication 3, **caractérisée en ce que** le guide (74) présente sur la face intérieure (73) du volet (7) dans la position finale définie un dispositif d'arrêt et/ou un fin de course (76) électrique pour la tablette (10).

7. Armoire selon l'une quelconque des revendications 2 à 5, **caractérisée en ce que** le joint (8) s'étendant le long du bord du volet (7) présente une arête d'étanchéité (81) fermée.

8. Procédé pour traiter le au moins un récipient (11, 12) qui contient un liquide (12, 13) à agiter, avec ou sans culture, lequel récipient se trouvant dans une armoire (5) qui a la forme d'un incubateur ou d'un réacteur et est fermée au moyen d'une porte (7), et étant maintenu dans un dispositif de serrage (19), l'armoire (5) présentant une tablette (10) disposée à l'intérieur, entraînée au moyen d'un entraînement agitateur-secoueur (15) et pouvant être sortie de l'armoire (5) le long d'un guide (74) avec le au moins un récipient (11, 12) se trouvant dessus, **caractérisé en ce qu'**une commande séquentielle (20) entraînant les étapes de procédé suivantes est prévue, **en ce que** la tablette (10) est arrêtée après la fin de l'opération d'agitation au moyen d'un dispositif de positionnement d'arrêt (16) dans une position définie prédéterminable, **en ce que** l'entraînement agitateur-secoueur (15) est désaccouplé, avant la sortie de la tablette (10), de celle-ci au moyen d'un dispositif d'accouplement (21), **en ce que**, ensuite et après l'ouverture de la porte (7), la tablette (10) est sortie de l'armoire (5) avec la porte ouverte (7) avec un entraînement de coulissement (18) le long du guide (74) jusqu'à atteindre une position finale définie, dans laquelle celle-ci est bloquée, et ensuite le au moins un récipient (11, 12) est traité automatiquement, de préférence par un robot, en utilisant une technologie des procédés.

9. Procédé selon la revendication 8, **caractérisé en ce que** la tablette (10) est retirée après ledit traitement par l'entraînement de coulissement (18) à nouveau à l'intérieur (9) de l'armoire (5).

10. Procédé selon la revendication 8 ou 9, **caractérisé en ce que** la tablette (10) est accouplée à l'entraînement agitateur-secoueur (15) au moyen du dispositif d'accouplement (21) après sa rentrée à l'intérieur (9) de l'armoire (5), après la fermeture de la porte (7) et avant le début de l'opération d'agitation.

11. Procédé selon l'une quelconque des revendications 8 à 10, **caractérisé en ce que,** après l'accouplement de la tablette (10) à l'entraînement agitateur-secoueur (15), celle-ci est mis à nouveau en mode d'agitation par la commande séquentielle (20) et le au moins un récipient (11, 12) est à nouveau agité.
